# EUROPEAN PATENT APPLICATION

(11) **EP 3 901 265 A1**
(43) Date of publication of application: **27.10.2021**
(21) Application number: 20826047.1
(22) Date of filing: 17.06.2020
(51) Int. Cl.: C12N 15/63, C12N 15/71, C12N 9/02, C12P 13/22

(54) **MICROORGANISMS FOR PRODUCING L-TYROSINE AND METHOD FOR PRODUCING L-TYROSINE BY USING SAME**

(30) Priority: 17.06.2019 KR 20190071797
(71) Applicant: CJ Cheiljedang Corporation, Seoul 04560 (KR)
(72) Inventor: SONG, Gyuhyeon, Seoul 04560 (KR); SEO, Chang Il, Seoul 04560 (KR); KWON, Nara, Seoul 04560 (KR)
(74) Representative: Mewburn Ellis LLP
(86) International application number: PCT/KR2020/007858
(87) International publication number: WO 2020/256415

(57) **Abstract**

The present disclosure relates to an L-tyrosine-producing microorganism, comprising a trp operon regulatory region and a gene encoding prephenate dehydratase operably linked thereto, and a method for producing L-tyrosine using the microorganism.

## Description

### [Technical Field]

The present disclosure relates to an L-tyrosine-producing microorganism, including a trp operon regulatory region and a gene encoding prephenate dehydratase operably linked thereto, and a method for producing L-tyrosine using the microorganism.

### [Background Art]

L-tyrosine is one of amino acids, and is used as an important material for pharmaceutical raw materials, food additives, animal feed, nutritional supplements, *etc.* In order to produce the L-tyrosine and other useful materials, various studies are underway to develop microorganisms with high-efficiency production and technologies for fermentation processes.

The production process of L-tyrosine by microorganisms starts with 3-deoxy-D-arobino-heptulosonate-7-phosphate (DAHP) produced by the polymerization reaction of phosphoenolpyruvate (PEP), which is an intermediate of glycolysis, with erythrose-4-phosphate (E4P), which is an intermediate of the pentose phosphate pathway. Then, DAHP is biosynthesized from chorismate to prephenate through the common aromatic biosynthetic pathway and is finally converted to L-tyrosine through the L-tyrosine biosynthetic pathway. During this process, chorismate can be branched into L-tryptophan, and prephenate can be branched into L-tyrosine or L-phenylalanine. Thus, when the common aromatic biosynthetic pathway is strengthened so as to increase the amount of L-tyrosine produced, it can be expected that the production of L-tryptophan and L-phenylalanine will also increase at the same time. That is, in order to produce L-tyrosine, phenylalanine and tryptophan are produced together as by-products, and accordingly, various studies such as gene recombination, purification, *etc.* must be carried out. Meanwhile, it is known that the production of L-tryptophan is regulated by repressors and attenuators according to the concentration of L-tryptophan produced by microorganisms (Korean Patent No. 10-0792095 B1).

Under these circumstances, the present inventors have made extensive efforts to develop a microorganism capable of producing L-tyrosine with high efficiency, and as a result, they have confirmed that when L-phenylalanine-producing genes are regulated by promoters of an L-tryptophan-producing operon, the production yield of L-tyrosine increases to a high level, thereby completing the present disclosure.

### [Disclosure]

### [Technical Problem]

One object of the present disclosure is to provide an L-tyrosine-producing microorganism, including a trp operon regulatory region and a gene encoding prephenate dehydratase operably linked thereto.

Another object of the present disclosure is to provide a method for producing L-tyrosine, including culturing the microorganism in a medium; and recovering L-tyrosine from the cultured microorganism or the medium.

Still another object of the present disclosure is to provide an expression cassette including a trp operon regulatory region and a gene encoding prephenate dehydratase operably linked thereto.

Still another object of the present disclosure is to provide a method for regulating the activity of prephenate dehydratase using a trp operon regulatory region and a gene encoding prephenate dehydratase operably linked thereto.

Still another object of the present disclosure is to provide the use of the L-tyrosine-producing microorganism for L-tyrosine production.

Still another object of the present disclosure is to provide the use of the expression cassette for L-tyrosine production.

Still another object of the present disclosure to provide the use of a composition for L-tyrosine production.

### [Advantageous Effects]

The L-tyrosine-producing microorganism of the present disclosure, which includes the gene encoding prephenate dehydratase and the trp operon regulatory region, minimizes the accumulation of L-phenylalanine while having no effect on the cell growth and can produce L-tyrosine with high efficiency.

### [Best Mode for Carrying Out the Invention]

The present disclosure is described in detail as follows. Meanwhile, respective descriptions and embodiments disclosed in the present disclosure may also be applied to other descriptions and embodiments. That is, all combinations of various elements disclosed in the present disclosure fall within the scope of the present disclosure. Further, the scope of the present disclosure cannot be considered to be limited by the specific description below.

To achieve the above objects, an aspect of the present disclosure provides an L-tyrosine-producing microorganism, including a trp operon regulatory region and a gene encoding prephenate dehydratase operably linked thereto.

As used herein, the term "L-tyrosine" is one of the 20 α-amino acids, and is classified as a hydrophilic amino acid or an aromatic amino acid. Tyrosine is a commercially critical amino acid used as a precursor of pharmaceuticals, flavonoids, alkaloids, *etc.*

As used herein, the term "tryptophan operon (Trp operon)" refers to a group of genes encoding enzymes involved in synthesizing tryptophan from chorismic acid (chorismate), and includes structural genes and expression regulatory regions (or regulatory regions). Specifically, the tryptophan operon may be a tryptophan operon derived from a microorganism of the genus *Corynebacterium* or a tryptophan operon derived from a microorganism of the genus *Escherichia,* but may include a tryptophan operon of various origin without limitation as long as it can regulate the *pheA* gene of the present disclosure. Specifically, the microorganism of the genus *Corynebacterium* may be *Corynebacterium glutamicum,* and the microorganism of the genus *Escherichia* may be *E. coli,* but the microorganisms are not limited thereto. Additionally, the tryptophan operon may have a known nucleotide sequence, and those skilled in the art can easily obtain the nucleotide sequence of the tryptophan operon from a database, such as NCBI or Kegg, *etc.* The common tryptophan operon is actively transcribed so as to produce a sufficient amount of tryptophan required by cells, but when sufficient tryptophan is present in the cells, a repressor binds to tryptophan and inactivates the tryptophan operon, thereby inhibiting transcription. In the present disclosure, the above term may be used interchangeably with tryptophan operon, trp operon, *etc.*

As used herein, the term "trp operon regulatory region" refers to a region that exists upstream of structural genes constituting the trp operon and can regulate the expression of the structural genes. The structural genes constituting the trp operon in the microorganism of the genus *Corynebacterium* may be composed of *trpE, trpG, trpD, trpC, trpB,* and *trpA* genes, and the structural genes constituting the trp operon in the microorganism of the genus *Escherichia* may be composed of *trpE, trpD, trpC, trpB,* and *trpA* genes. The trp operon regulatory region may exist upstream of trpE at the 5' position of the trp operon structural genes. Specifically, it may include a trp regulator (trpR), a promoter (trp promoter), an operator (trp operator), a trp leader peptide (trp L), and a trp attenuator excluding the structural genes that may constitute the trp operon. More specifically, it may include a promoter (trp promoter), an operator (trp operator), a trp leader peptide (trp L), and a trp attenuator (trp attenuator). In the present disclosure, the trp operon regulatory region may include, without limitation, any regulatory region located upstream of the *pheA* gene encoding the prephenate dehydratase and can regulate the expression of the *pheA* gene. In the present disclosure, the trp operon regulatory region may be used interchangeably with a trp operon regulatory factor, a trpE promoter, a trp operon promoter, a trpE regulatory region, a trpE regulatory factor, and a trpE regulatory sequence.

For example, the trp operon regulatory region may include a nucleotide sequence of SEQ ID NO: 1, but is not limited thereto. The sequence of SEQ ID NO: 1 can be confirmed from GenBank of NCBI, a known database.

Specifically, the trp operon regulatory region may consist of the nucleotide sequence of SEQ ID NO: 1 or a nucleotide sequence having a homology or identity of at least 70%, 80%, 85%, 90%, 95%, 96%, 97%, 98%, or 99% or more to SEQ ID NO: 1. Additionally, it is apparent that an regulatory region with deletion, modification, substitution, or addition of a part of the sequence also falls within the scope of the present disclosure as long as its nucleotide sequence has such a homology or identity and has a function corresponding to the regulatory region.

As used herein, the term "homology" and "identity" refer to the degree of relevance between two given amino acid sequences or nucleotide sequences, and may be expressed as a percentage. The terms "homology" and "identity" are often used interchangeably with each other.

The sequence homology or identity of conserved polynucleotides or polypeptides may be determined by standard alignment algorithms and can be used together with default gap penalty established by the program being used. Substantially, homologous or identical sequences are generally expected to hybridize to all or at least about 50%, about 60%, about 70%, about 80%, about 85% or about 90% of the entire length of the sequences under moderate or high stringent condition. Polynucleotides that contain degenerate codons instead of codons are also considered in the hybridization of the polynucleotides.

The homology or identity of the polypeptide or polynucleotide sequences may be determined by, for example, BLAST algorithm by literature [see: Karlin and Altschul, Pro. Natl. Acad. Sci. USA, 90, 5873(1993)], or FASTA by Pearson (see: Methods Enzymol., 183, 63, 1990). Based on the algorithm BLAST, a program referred to as BLASTN or BLASTX has been developed (see: www.ncbi.nlm.nih.gov). Further, whether any amino acid or polynucleotide sequences have a homology, similarity, or identity with each other, it may be identified by comparing the sequences in a Southern hybridization experiment under stringent conditions as defined, and appropriate hybridization conditions defined are within the skill of the art, and may be determined by a method well known to those skilled in the art (for example, J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology*)*

As used herein, the term "prephenate dehydratase" is one of the proteins essential for the biosynthesis of L-phenylalanine. The gene encoding the protein may be, for example, a *pheA* gene, but is not limited thereto. The protein may also be referred to as a bifunctional chorismate mutase/prephenate dehydratase. Since the prephenate dehydratase is an enzyme in the pathway that produces L-phenylalanine from chorismate or prephenate, and is an enzyme in the step of competing with the tyrosine biosynthetic pathway, it is generally selected as a target gene for inactivating the production of tyrosine-producing strains. However, when the prephenate dehydratase is inactivated, there is a problem in that the productivity is greatly reduced as it has an effect on the growth of the strains.

In the present disclosure, the microorganisms that have been genetically modified such that the *pheA* gene was regulated by the trp operon regulatory region, specifically, the microorganisms in which the regulatory region of the trp operon was applied to the promoter region for the regulation of pheA minimize the accumulation of phenylalanine while having no effect on the cell growth and is effective in improving tyrosine production.

Additionally, the *pheA* gene, which is a gene encoding the prephenate dehydratase, may have a deletion in a part or all of the regulatory sequence existing upstream of the *pheA* structural gene. For the purpose of the present disclosure, the upstream region of the *pheA* structural gene may be substituted by the trp operon regulatory region, or the trp operon regulatory region may be inserted into the upstream region of the *pheA* structural gene to include the *pheA* structural gene which can be regulated by the expression of protein encoded by *pheA* gene by the trp operon regulatory region. As used herein, the term *"pheA* gene" may be used interchangeably with "the gene encoding prephenate dehydratase" and *"the pheA* structural gene".

As used herein, the term "L-tyrosine-producing microorganism" refers to a microorganism naturally having an L-tyrosine-producing ability, or a microorganism acquired with an L-tyrosine-producing ability from its parent strain having no L-tyrosine-producing ability. Specifically, the microorganism may be an L-tyrosine-producing microorganism including a trp operon regulatory region and a gene encoding prephenate dehydratase operably linked thereto, but is not limited thereto.

Specifically, the "L-tyrosine-producing microorganism" includes all of wild-type microorganisms and naturally or artificially genetically modified microorganisms, and it may be a microorganism having a genetic modification or enhanced activity for a desired L-tyrosine production, which may be a microorganism in which a particular mechanism is attenuated or reinforced mechanism due to insertion of a foreign gene, or reinforcement or inactivation of activity of an endogenous gene. For the purpose of the present disclosure, the L-tyrosine-producing microorganism includes the trp operon regulatory region and the gene encoding prephenate dehydratase operably linked thereto, and has an increased L-tyrosine-producing ability, and may be a genetically modified microorganism or a recombinant microorganism, but is not limited thereto.

More specifically, the microorganism having an L-tyrosine-producing ability in the present disclosure refers to a recombinant microorganism having an enhanced L-tyrosine-producing ability by including the trp operon regulatory region and the gene encoding prephenate dehydratase operably linked thereto of the present disclosure, or by being transformed into a vector including the trp operon regulatory region and the gene encoding prephenate dehydratase operably linked thereto.

In the present disclosure, the "microorganism including a gene" may mean "a genetically modified microorganism", "a microorganism modified to express a gene", "a recombinant microorganism expressing a gene", or "a recombinant microorganism having the activity of a protein encoded by a gene", but is not limited thereto.

For example, it may be i) a microorganism including the trp operon regulatory region and the gene encoding prephenate dehydratase operably linked thereto of the present disclosure, ii) a microorganism which is modified to express the trp operon regulatory region and the gene encoding prephenate dehydratase operably linked thereto, iii) a recombinant microorganism expressing the trp operon regulatory region and the gene encoding prephenate dehydratase operably linked thereto, and iv) a recombinant microorganism which is transformed into a vector including the trp operon regulatory region and the gene encoding prephenate dehydratase operably linked thereto to have an enhanced L-tyrosine-producing ability, but is not limited thereto.

The "microorganism having an enhanced L-tyrosine-producing ability" may refer to a microorganism having an enhanced L-tyrosine-producing ability compared to the parent strain or a non-modified microorganism before transformation. The "non-modified microorganism" may be a native strain itself or a microorganism which has not been transformed into a vector including the genes encoding the polynucleotide and the target protein.

As the microorganism for the purpose of the present disclosure, all microorganisms capable of producing L-tyrosine by including the trp operon regulatory region and the gene encoding prephenate dehydratase operably linked thereto may be included. In the present disclosure, "the microorganism capable of producing L-tyrosine" may be interchangeably used with "the L-tyrosine-producing microorganism", and "the microorganism having an L-tyrosine-producing ability".

The microorganism may be, for example, a microorganism belonging to the genus *Enterbacter,* a microorganism belonging to the genus *Escherichia,* a microorganism belonging to the genus *Erwinia,* a microorganism belonging to the genus *Serratia,* a microorganism belonging to the genus *Providencia,* a microorganism belonging to the genus *Corynebacterium,* and a microorganism belonging to the genus *Brevibacterium,* but is not limited thereto.

Specifically, the microorganism may be a microorganism belonging to the genus *Corynebacterium.* In the present disclosure, "the microorganism belonging to the genus *Corynebacterium"* may specifically be *Corynebacterium glutamicum, Corynebacterium ammoniagenes, Brevibacterium lactofermentum, Brevibacterium flavum, Corynebacterium thermoaminogenes, Corynebacterium efficiens, Corynebacterium stationis, etc.,* but is not limited thereto.

Another aspect of the present disclosure provides a method for producing L-tyrosine, including culturing the microorganism in a medium; and recovering L-tyrosine from the cultured microorganism or the medium.

In the method above, the step of culturing the microorganism may be performed by a known batch culture method, continuous culture method, fed-batch culture method, *etc.,* but is not particularly limited thereto. In particular, with respect to the culture conditions, the pH of the culture may be adjusted to a suitable pH *(e.g.,* pH 5 to 9, specifically pH 6 to 8, and most specifically pH 7.0) using a basic compound (e.g., sodium hydroxide, potassium hydroxide, or ammonia) or acidic compound (e.g., phosphoric acid or sulfuric acid), but is not particularly limited thereto. Additionally, oxygen or an oxygen-containing gas mixture may be injected into the culture in order to maintain an aerobic state. The culture temperature may be maintained at 20°C to 45°C, specifically at 25°C to 40°C, and the culturing may be performed for about 10 hours to 160 hours, but the culture is not limited to the above. The amino acid produced by the culture may be secreted into the medium or may remain in the cells.

Further, as a carbon source for the culture medium to be used, sugars and carbohydrates *(e.g.,* glucose, sucrose, lactose, fructose, maltose, molasses, starch, and cellulose), oils and fats *(e.g.,* soybean oil, sunflower seed oil, peanut oil, and coconut oil), fatty acids *(e.g.,* palmitic acid, stearic acid, and linoleic acid), alcohols *(e.g.,* glycerol and ethanol), and organic acids *(e.g.,* acetic acid) may be used alone or in combination, but the carbon source is not limited thereto. As a nitrogen source, nitrogen-containing organic compounds (e.g., peptone, yeast extract, meat gravy, malt extract, corn steep liquor, soybean flour, and urea) or inorganic compounds (e.g., ammonium sulfate, ammonium chloride, ammonium phosphate, ammonium carbonate, and ammonium nitrate) may be used alone or in combination, but the nitrogen source is not limited thereto. As a phosphorus source, potassium dihydrogen phosphate, dipotassium hydrogen phosphate, corresponding sodium-containing salts thereof, *etc.* may be used alone or in combination, but the phosphorus source is not limited thereto. In addition, essential growth-promoting materials such as other metal salts (e.g., magnesium sulfate or iron sulfate), amino acids, and vitamins may be contained in the medium.

In the method for recovering the amino acid produced in the culturing step of the present disclosure, the target amino acids may be collected from the cultured solution using an appropriate method known in the art according to the culture method. For example, centrifugation, filtration, anion-exchange chromatography, crystallization, HPLC, *etc.* may be used, and the target amino acids may be recovered from the medium or the microorganism using an appropriate method known in the art.

Additionally, the recovery step may further include a purification process, and it may be performed using an appropriate method known in the art. Thus, the recovered amino acids may be in a purified state or in the form of a microbial fermentation solution containing the amino acids *(*Introduction to Biotechnology and Genetic Engineering, A. J. Nair., 2008). Further, before and after the culture step, and before and after the recovery step, the target amino acids may be efficiently recovered by further carrying out an appropriate method known in the art.

Still another aspect of the present disclosure provides an expression cassette including the trp operon regulatory region and the gene encoding prephenate dehydratase operably linked thereto.

In the present disclosure, the term "expression cassette" may be a gene construct unit including an essential regulatory element operably linked to the gene so that the introduced gene is expressed when it is present in the cells of an individual. The expression cassette may be, for example, in the form of an expression vector, but is not limited thereto, and may include all gene constructs having the smallest unit capable of expressing the target gene to be introduced.

The expression cassette may be prepared and purified by a standard recombinant DNA technology. Any type of the expression cassette may be used without particular limitation as long as it can function to express a desired gene in various host cells such as prokaryotic cells and eukaryotic cells and as long as it can function to produce a desired protein. The expression cassette may include a promoter, a start codon, a gene encoding a target protein, or a stop codon. In addition, it may appropriately include a DNA encoding a signal peptide, an enhancer sequence, untranslated regions at the 5' and 3' ends of a target gene, a selectable marker region, or a replicable unit, *etc.* Moreover, the expression cassette may include a mono-cistronic vector including a polynucleotide encoding one protein, or a poly-cistronic vector including a polynucleotide encoding two or more recombinant proteins, but is not limited thereto.

As used herein, the term "vector" may refer to a DNA construct containing the nucleotide sequence of a polynucleotide encoding the target protein, which is operably linked to a suitable regulatory sequence such that the target protein can be expressed in an appropriate host. The regulatory sequence may include a promoter capable of initiating transcription, any operator sequence for regulating the transcription, a sequence encoding an appropriate mRNA ribosome-binding domain, and a sequence regulating the termination of transcription and translation. After being transformed into a suitable host cell, the vector may be replicated or function irrespective of the host genome, and may be integrated into the host genome itself.

The vector used in the present disclosure is not particularly limited as long as it can be expressed in a host cell, and any vector known in the art may be used. Examples of conventionally used vectors may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, Charon21A, *etc.,* may be used; and as a plasmid vector, those based on pBR, pUC, pBluescriptII, pGEM, pTZ, pCL, pET, *etc.* may be used. Specifically, the vectors pDZ, pACYC177, pACYC184, pCL, pECCG117, pUC19, pBR322, pMW118, pCC1BAC, *etc.* may be used, but is not limited thereto.

The vectors which can be used in the present disclosure are not particularly limited, and a known expression vector can be used. Additionally, a gene or polynucleotide encoding a target protein can be inserted into a chromosome through a vector for intracellular chromosomal insertion. The insertion of the polynucleotide into the chromosome may be performed by any methods known in the art, for example, by homologous recombination, but is not limited thereto. In addition, the vector may further include a selection marker for confirming the insertion into the chromosome. The selection marker is used to select the cells which have been transformed into vectors, *i.e.,* to confirm whether the target polynucleotide has been inserted, and markers which can show drug resistance, auxotrophy, resistance to cytotoxic agents, or provide selectable phenotypes such as the expression of surface proteins may be used. Under the circumstances where selective agents are treated, only the cells capable of expressing the selection markers can survive or express other phenotypic traits, and thus the transformed cells can be selected.

As used herein, the term "transformation" refers to the introduction of a vector including a polynucleotide encoding a target polypeptide into a host cell in such a way that the protein encoded by the polynucleotide is expressed in the host cell. As long as the transformed polynucleotide can be expressed in the host cell, it can be either inserted into the chromosome of the host cell and located therein, or located extrachromosomally, and both cases may be included. Additionally, the polynucleotide includes DNA and RNA which encode the target protein. The polynucleotide may be introduced in any form as long as it can be introduced into a host cell and expressed therein. For example, the polynucleotide may be introduced into a host cell in the form of an expression cassette, which is a gene construct including all elements necessary for self-expression. The transformation method includes any method of introducing a nucleic acid into a cell, and may be carried out by selecting a suitable standard technique known in the art, depending on a host cell. For example, the transformation may be carried out via electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, *etc.,* but the method is not limited thereto.

Further, as used herein, the term "operably linkage" refers to a functional linkage between the above polynucleotide sequence with a promoter sequence, which initiates and mediates the transcription of the polynucleotide encoding the target protein of the present disclosure, or an regulatory region. The operable linkage may be prepared by a gene recombination technique known in the art, and site-specific DNA cleavage and ligation may be performed using known lyase, ligase, *etc.,* but these are not limited thereto.

Still another aspect of the present disclosure provides a composition for producing L-tyrosine, including the trp operon regulatory region and the gene encoding prephenate dehydratase operably linked thereto.

The composition for producing L-tyrosine includes the trp operon regulatory region and the gene encoding prephenate dehydratase operably linked thereto, and may further include without limitation the configuration capable of operating the gene or the operon regulatory region. The gene encoding the prephenate dehydratase and the trp operon regulatory region may be in a form included within a vector so that a gene operably linked in the introduced host cell can be expressed. The expression of the pheA gene, which is a gene encoding the prephenate dehydratase, may be regulated by the trp operon regulatory region.

Still another aspect of the present disclosure provides a method for regulating the activity of prephenate dehydratase using the trp operon regulatory region and the gene encoding prephenate dehydratase operably linked thereto.

Still another aspect of the present disclosure provides the use of the L-tyrosine-producing microorganism for the production of L-tyrosine.

Still another aspect of the present disclosure provides the use of the expression cassette for the production of L-tyrosine.

Still another aspect of the present disclosure provides the use of the composition for the production of L-tyrosine.

### [Mode for Carrying Out the Invention]

Hereinafter, the present disclosure will be described in more detail with reference to the following Examples. However, these Examples are for illustrative purposes only and the scope of the invention is not limited by these Examples.

### Example 1: Construction of L-Tyrosine-Producing Strain

Although the wild-type *Corynebacterium glutamicum* has the ability to produce L-tyrosine, it does not produce L-tyrosine in excess to be released into a cultured medium. According to the purpose of the present disclosure, in order to identify a genetic trait that increases the L-tyrosine-producing ability, a strain with an increased L-tyrosine-producing ability was used rather than the wild-type strain. Therefore, an L-tyrosine-producing strain was constructed by enhancing the genes necessary to produce L-tyrosine based on the *Corynebacterium glutamicum* ATCC 13869 strain.

First, for the enhanced supply of erythrose 4 phosphate (E4P) as a precursor of L-tyrosine, *tkt* genes were overexpressed. At the same time, *aroP,* an aromatic amino acid importer gene that introduces L-tyrosine into the cells, was deleted.

For the genetic manipulation, downstream and upstream regions of the *aroP* gene into which the *tkt* gene was to be inserted by substitution were first obtained. Specifically, a gene fragment in the downstream region of the *aroP* gene was obtained using the primers of SEQ ID NO: 2 and SEQ ID NO: 3, and a gene fragment in the upstream region of the *aroP* gene was obtained using the primers of SEQ ID NO: 4 and SEQ ID NO: 5 based on the *Corynebacterium glutamicum* ATCC13869 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 60 seconds, and then polymerization at 72°C for 5 minutes.

Additionally, in order to obtain the *tkt* gene including the tkt promoter, a *tkt* gene fragment including the tkt promoter was obtained using the primers of SEQ ID NO: 6 and SEQ ID NO: 7 based on the *Corynebacterium glutamicum* ATCC13869 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 150 seconds, and then polymerization at 72°C for 5 minutes.

The amplified upstream and downstream regions of the aroP promoter, the *tkt* gene fragment including the tkt promoter, and the vector pDZ (Korean Patent No. 10-0924065) for chromosomal transformation cleaved by *Sma*I restriction enzyme were cloned using the Gibson assembly method (DG Gibson et al., NATURE METHODS, VOL.6 NO.5, MAY 2009, NEBuilder HiFi DNA Assembly Master Mix) to obtain a recombinant plasmid, which was named pDZ-ΔaroP: :Pn-tkt. The cloning was performed by mixing the Gibson assembly reagent and each of the gene fragments in a calculated number of moles followed by incubating at 50°C for 1 hour.

Primer sequences used to construct each of the vectors are shown in Table 1 below.

**[Table 1]**

| SEQ ID NO: | Sequence (5'-3') |
|---|---|
| 2 | TCGAGCTCGGTACCCTGGGAACTTGTCGACGCTAT |
| 3 | TGTTCGGCAAGCATTGTGGTGTGGGCAATGATCAC |
| 4 | ATTAACGGTTAAAGTACTCATTGTGAGGTGGCGGG |
| 5 | CTCTAGAGGATCCCCGGAGCTGCTGTCCAACGTGG |
| 6 | CCACACCACAATGCTTGCCGAACATTTTTCTTTTC |
| 7 | CACAATGAGTACTTTAACCGTTAATGGAGTCCTTG |

The constructed pDZ-AaroP::Pn-tkt vector was transformed into the *Corynebacterium glutamicum* ATCC 13869 strain by electroporation and then subjected to a secondary crossover to obtain a strain into which the *tkt* gene including the tkt promoter was inserted, while deleting the *aroP* gene at the same time. The corresponding genetic manipulation was confirmed through genome sequencing and a PCR method using the primers of SEQ ID NO: 8 and SEQ ID NO: 9, which can respectively amplify the external region of the upstream region and downstream region of the homologous recombination where the corresponding gene was inserted, and the resulting strain was named CM06-0001.

**[Table 2]**

| SEQ ID NO: | Sequence (5'-3') |
|---|---|
| 8 | ACGCGCCAAGTCGGACG |
| 9 | CGCACGATGTTTACCTGCG |

In order to strengthen the L-tyrosine pathway, a *tyrA* gene that receives a feedback regulation by L-tyrosine possessed by *Corynebacterium glutamicum* was replaced with a variant *tyrA* that does not receive the feedback regulation derived from *E. coli* including a strong gapA promoter. It is known that in the *E.* coli-derived tyrA protein, the feedback is released when methionine at position 53 is mutated to isoleucine, and alanine at position 354 is mutated to valine, and this form of protein (SEQ ID NO: 10) was used *(*Appl. Microbiol. Biotechnol. 75, 103-110 (2007)).

For the genetic manipulation, upstream and downstream regions of the *tyrA* gene in which the *tyrA* gene was to be inserted by substitution were first obtained. Specifically, a gene fragment in the upstream region of the *tyrA* gene was obtained using the primers of SEQ ID NO: 11 and SEQ ID NO: 12, and a gene fragment in the downstream region of the *tyrA* gene was obtained using the primers of SEQ ID NO: 13 and SEQ ID NO: 14 based on the *Corynebacterium glutamicum* ATCC13869 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 60 seconds, and then polymerization at 72°C for 5 minutes.

Additionally, in order to obtain an E. coli-derived variant *tyrA* gene including a gapA promoter, a gapA promoter fragment was obtained using the primers of SEQ ID NO: 15 and SEQ ID NO: 16 based on the *Corynebacterium glutamicum* ATCC13869 chromosomal DNA as a template through PCR, and an E. coli-derived variant *tyrA* gene fragment was obtained using the primers of SEQ ID NO: 17 and SEQ ID NO: 18 based on the *E.* coli-derived variant tyrA synthetic DNA as a template through PCR.

Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 60 seconds, and then polymerization at 72°C for 5 minutes.

The amplified upstream and downstream regions of the *tyrA* gene, the *E.* coli-derived variant *tyrA* gene fragment including the gapA promoter, and the vector pDZ for chromosomal transformation cleaved by *Sma*I restriction enzyme were cloned using the Gibson assembly method to obtain a recombinant plasmid, which was named pDZ-ΔtyrA::PgapA-tyrAm. The cloning was performed by mixing the Gibson assembly reagent and each of the gene fragments in a calculated number of moles, followed by incubating at 50°C for 1 hour.

Primer sequences used to construct each of the vectors are shown in Table 3 below.

**[Table 3]**

| SEQ ID NO: | Sequence (5'-3') |
|---|---|
| 11 | TTCGAGCTCGGTACCCTATCAAAACCGAGTTCTTCC |
| 12 | GTCGTTTTTAGGCCTCCTGACAAGTGTGGCACATAC |
| 13 | TGACAATCGCCAGTAATTTTATCGGCTGATGATTCT |
| 14 | ACTCTAGAGGATCCCCAACGCGATTGCATTCGGCTC |
| 15 | GTGCCACACTTGTCAGGAGGCCTAAAAACGACCGAG |
| 16 | TCAATTCAGCAACCATGTTGTGTCTCCTCTAAAGAT |
| 17 | TTAGAGGAGACACAACATGGTTGCTGAATTGACCGC |
| 18 | TCATCAGCCGATAAAATTACTGGCGATTGTCATTCG |

The constructed pDZ-ΔtyrA::PgapA-tyrAm vector was transformed into the CM06-0001 strain by electroporation and then subjected to a secondary crossover to obtain a strain in which the *E.* coli-derived variant *tyrA* gene including the gapA promoter was inserted, while deleting the *tyrA* gene at the same time. The corresponding genetic manipulation was confirmed through genome sequencing and a PCR method using the primers of SEQ ID NO: 19 and SEQ ID NO: 20, which can respectively amplify the external region of the upstream region and downstream region of the homologous recombination where the corresponding gene was inserted, and the resulting strain was named CM06-0002.

**[Table 4]**

| SEQ ID NO: | Sequence (5'-3') |
|---|---|
| 19 | GCCCACTAGTCGAATCCC |
| 20 | CTGTCCGCAACCTGTGCG |

In order to increase L-tyrosine production, the *aroG* gene involved in the first step of the common aromatic biosynthetic pathway was enhanced by adding a strong promoter to an *E*. coli-derived feedback regulation release variant *aroG.* It is known that in the *E.* coli-derived aroG protein, the feedback is released when proline at position 150 is substituted with leucine, and this form of protein (SEQ ID NO: 68) was used *(*Appl. Environ. Microbiol. 63, 761-762 (1997)).

For the genetic manipulation, downstream and upstream regions into which the *aroG* gene was to be further inserted were obtained. Specifically, a gene fragment in the upstream region of BBD29_14470 gene was obtained using the primers of SEQ ID NO: 21 and SEQ ID NO: 22, and a gene fragment in the downstream region of BBD29_14470 gene was obtained using the primers of SEQ ID NO: 23 and SEQ ID NO: 24 based on the *Corynebacterium glutamicum* ATCC13869 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 60 seconds, and then polymerization at 72°C for 5 minutes.

The amplified upstream and downstream regions in which the variant *aroG* was to be further inserted, and the vector pDZ for chromosomal transformation cleaved by *Sma*I restriction enzyme were cloned using the Gibson assembly method to obtain a recombinant plasmid, which was named pDZ-Δ BBD29_14470. The cloning was performed by mixing the Gibson assembly reagent and each of the gene fragments in a calculated number of moles, followed by incubating at 50°C for 1 hour.

Additionally, in order to obtain an *E*. coli-derived variant *aroG* gene including a gapA promoter, a gapA promoter fragment was obtained using the primers of SEQ ID NO: 15 and SEQ ID NO: 26 based on the *Corynebacterium glutamicum* ATCC13869 chromosomal DNA as a template through PCR, and an *E*. coli-derived variant *aroG* gene fragment was obtained using the primers of SEQ ID NO: 27 and SEQ ID NO: 28 based on the *E.* coli-derived feedback release variant aroG synthetic DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 60 seconds, and then polymerization at 72°C for 5 minutes.

The amplified variant *aroG* gene fragment including the gapA promoter, and the vector pDZ-ΔBBD29_14470 for chromosomal transformation cleaved by *Sca*I restriction enzyme were cloned using the Gibson assembly method to obtain a recombinant plasmid, which was named pDZ-ΔBBD29_14470::PgapA-aroGm. The cloning was performed by mixing the Gibson assembly reagent and each of the gene fragments in a calculated number of moles, followed by incubating at 50°C for 1 hour.

Primer sequences used to construct each of the vectors are shown in Table 5 below.

**[Table 5]**

| SEQ ID NO: | Sequence (5'-3') |
|---|---|
| 21 | TCGAGCTCGGTACCCCCCGGCGGTATCGAGGTAGT |
| 22 | GACAAGTTTAGTACTTTAATCACCCGCGGGGACCC |
| 23 | GGTGATTAAAGTACTAAACTTGTCCCGAGGGTGAG |
| 24 | CTCTAGAGGATCCCCTATCAGTCACTTCCCTGAGA |
| 25 | GCGGGTGATTAAAGTGAGGCCTAAAAACGACCGAG |
| 26 | GTTCTGATAATTCATGTTGTGTCTCCTCTAAAGAT |
| 27 | ATGAATTATCAGAACGACGA |
| 28 | CGGGACAAGTTTAGTTTACCCGCGACGCGCTTTTA |

The constructed pDZ-ΔBBD29_14470::PgapA-aroGm vector was transformed into the CM06-0002 strain by electroporation and then subjected to a secondary crossover to obtain a strain into which the *E.* coli-derived feedback release variant aroG gene including the gapA promoter was inserted. The corresponding genetic manipulation was confirmed through genome sequencing and a PCR method using the primers of SEQ ID NO: 29 and SEQ ID NO: 30, which can respectively amplify the external region of the upstream region and downstream region of the homologous recombination where the corresponding gene was inserted, and the resulting strain was named CM06-0003.

**[Table 6]**

| SEQ ID NO: | Sequence (5'-3') |
|---|---|
| 29 | TTGATATGACCGCAGCCTGA |
| 30 | CTGCATTCTCATCGATCTTG |

### Example 2: Evaluation of Production Ability of L-Tyrosine-Producing Strains

In order to confirm the L-tyrosine-producing ability of the strains constructed in Example 1, the strains were cultured and evaluated in the following manner. Each of the strains was inoculated into a 250-mL corner-baffled flask containing 25 mL of the following seed medium and was cultured with shaking at 200 rpm at 30°C for 20 hours. Next, 1 mL of the seed culture solution was inoculated into a 250-mL corner-baffled flask containing 25 mL of the following production medium and was cultured with shaking at 200 rpm at 30°C for 24 hours. After the culture, the production amount of L-tyrosine, L-phenylalanine, and L-tryptophan was measured by HPLC.

### <Seed medium (pH 7.0) >

20 g glucose, 10 g peptone, 5 g yeast extract, 1.5 g urea, 4 g KH₂PO₄, 8 g K₂HPO₄, 0.5 g MgSO₄.7H₂O, 100 µg biotin, 1,000 µg thiamine HCl, 2,000 µg calcium-pantothenate, and 2,000 µg nicotinamide (per liter of distilled water)

### <Production medium (pH 7.0) >

30 g glucose, 15 g (NH₄)₂SO₄, 1.2 g MgSO₄.7H₂O, 1 g KH₂PO₄, 5 g yeast extract, 900 µg biotin, 4,500 µg thiamine HCl, 4,500 µg calcium-pantothenate, and 30 g CaCO₃ (per liter of distilled water).

**[Table 7]**

| Strain No. | Genotype | Glucose used (g/L) | Production amount of L-tyrosine (g/L) | Production amount L-phenylalanine (g/L) | Production amount L-tryptophan (g/L) |
|---|---|---|---|---|---|
| ATCC 13869 | Wild-type | 30 | 0.00 | 0.00 | 0.00 |
| CM06-0001 | ATCC 13869 ΔaroP: :Pn-tkt | 30 | 0.00 | 0.00 | 0.00 |
| CM06-0002 | CM06-0001 ΔtyrA: :PgapA-tyrAm | 30 | 0.00 | 0.00 | 0.00 |
| CM06-0003 | CM06-0002 | 30 | 0.38 | 1.11 | 0.02 |
| | ΔBBD29 _14470: :PgapA-aro Gm | | | | |

The results of L-tyrosine, L-phenylalanine, and L-tryptophan production in the cultures of the wild-type *Corynebacterium glutamicum* ATCC 13869, CM06-0001, CM06-0002, and CM06-0003 are as shown in Table 7.

L-tyrosine was not produced in CM06-0001, which is a strain that enhances the supply of E4P, a precursor, prepared by deleting the aromatic amino acid importer gene from the wild-type strain, and L-tyrosine was also not detected in CM06-0002, in which the tyrA feedback inhibition was further released in CM06-0001.

The L-tyrosine and L-phenylalanine production was confirmed in the CM06-0003 strain, in which the common aromatic compounds production pathway enhanced by releasing the feedback inhibition of aroG in the CM06-0002 strain. Additionally, the L-tyrosine and L-phenylalanine production increased significantly compared to the previous strains, but the L-tryptophan production showed no dramatic change.

### Example 3: Deletion of pheA Gene in L-Tyrosine-Producing Strains

It was confirmed that when the common aromatic production pathway was enhanced in Example 2, L-phenylalanine was produced in the largest amount, followed by the production of L-tyrosine, and L-tryptophan was produced in a small amount. Therefore, it can be expected that the L-tyrosine and L-tryptophan production will increase if the L-phenylalanine production pathway is deleted.

In order to delete the L-phenylalanine production pathway, *pheA,* a gene involved in the first step of branching out from prephenic acid, was deleted. For such a gene deletion, the upstream and downstream regions of the *pheA* gene were first obtained. Specifically, a gene fragment in the upstream region of the *pheA* gene was obtained using the primers of SEQ ID NO: 31 and SEQ ID NO: 32, and a gene fragment in the downstream region of the *pheA* gene was obtained using the primers of SEQ ID NO: 33 and SEQ ID NO: 34 based on the *Corynebacterium glutamicum* ATCC13869 chromosomal DNA as a template through PCR. Solg™ Pfu-X DNA polymerase was used as the polymerase and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 60 seconds, and then polymerization at 72°C for 5 minutes.

The amplified upstream and downstream regions of the *pheA* gene, and the vector pDZ for chromosomal transformation cleaved by *Sma*I restriction enzyme were cloned using the Gibson assembly method to obtain a recombinant plasmid, which was named pDZ-ΔpheA. The cloning was performed by mixing the Gibson assembly reagent and each of the gene fragments in a calculated number of moles, followed by incubating at 50°C for 1 hour.

Primer sequences used to construct each of the vectors are shown in Table 8 below.

**[Table 8]**

| SEQ ID NO: | Sequence (5'-3') |
|---|---|
| 31 | TTCGAGCTCGGTACCCCCGACCAGGCCACACGCG |
| 32 | AATAATCCGGCCGGCCATGGGGTTACACAGCTTAA CCCGC |
| 33 | CGGGTTAAGCTGTGTAACCCCATGGCCGGCCGGAT TATT |
| 34 | CGACTCTAGAGGATCCCCGCTGACAACAGCAACGT CG |

The constructed pDZ-ΔpheA vector was transformed into the CM06-0003 strain by electroporation and then subjected to a secondary crossover to obtain a strain in which the *pheA* was deleted. The corresponding genetic manipulation was confirmed through genome sequencing and a PCR method using the primers of SEQ ID NO: 35 and SEQ ID NO: 36, which can respectively amplify the external region of the upstream region and downstream region of the homologous recombination where the corresponding gene was deleted, and the resulting strain was named CM06-0004.

**[Table 9]**

| SEQ ID NO: | Sequence (5'-3') |
|---|---|
| 35 | CCAGCGATGATCGCGCCG |
| 36 | ATCGCCGTGGAGCCAGCC |

### Example 4: Evaluation of Production Ability of L-Tyrosine-Producing Strains in which pheA Gene is deleted

In order to confirm the L-tyrosine-producing ability of the strains constructed in Example 3, the strains were cultured using the method and the medium compositions described in Example 2.

**[Table 10]**

| Strain No. | Genotype | Glucose used (g/L) | Production amount of L-tyrosine (g/L) | Production amount L-phenylalanine (g/L) | Production amount L-tryptophan (g/L) |
|---|---|---|---|---|---|
| CM06-0003 | ΔaroP: :Pn-tkt, ΔtyrA: :PgapA-tyrAm, ΔBBD29_14470::PgapA-ar oGm | 30 | 0.39 | 1.10 | 0.02 |
| CM06-0004 | CM06-0003 ΔpheA | 13 | 0.89 | 0.04 | 0.02 |

The results of L-tyrosine, L-phenylalanine, and L-tryptophan production in the cultures of L-tyrosine-producing strains CM06-0003 and CM06-0004 are shown in Table 10 above. The CM06-0004 strain, in which *pheA* is deleted, produced L-tyrosine as a major product with a significant decrease in L-phenylalanine production. As in Example 2, no significant change in L-tryptophan production was observed.

However, since the CM06-0004 strain could not produce L-phenylalanine, it could only grow depending on L-phenylalanine contained in the yeast extract of the medium, and showed only about 1/3 of the sugar consumption compared to the strain in which *pheA* is not deleted. That is, it was confirmed that when the *pheA* gene was deleted in microorganisms, it had a great effect on the growth of the strains, so that the strains could not be utilized for the purpose of producing the target product.

### Example 5: Construction of of L-Tyrosine-Producing Strains in which pheA Gene Promoter is substituted

From the results of Examples 2 and 4, it can be predicted that L-tryptophan concentration is precisely regulated such that the growth of cells is not interfered while L-tryptophan is maintained at a low concentration at all times during culture. It is also known in the literature that the L-tryptophan production is simultaneously regulated by attenuator and promoter according to L-tryptophan concentration *(*Appl. Environ Microbiol 59 791, 1993).

Therefore, the *pheA,* an L-phenylalanine-producing gene, was subjected to a regulatory mechanism of L-tryptophan, so that the *pheA* gene could be regulated according to the L-tryptophan concentration.

In particular, as a control, strains inserted with an ilvB promoter that allows *pheA* to be regulated by L-isoleucine concentration, and a leuA promoter and leuC promoter that allow *pheA* to be regulated by L-leucine concentration, in addition to the trpE regulatory region that allows *pheA* to be regulated by L-tryptophan concentration, were also constructed and compared.

In order to allow the *pheA* gene to be regulated by the regulatory region of *trpE* gene, an upstream region into which the gene was to be inserted, a trpE regulatory region, and a downstream region into which the gene was to be inserted were obtained. Specifically, a gene fragment in the upstream region into which the gene was to be inserted was obtained using the primers of SEQ ID NO: 37 and SEQ ID NO: 38; a gene fragment in the trpE regulatory region was obtained using the primers of SEQ ID NO: 39 and SEQ ID NO: 40; and a gene fragment in the downstream region into which the gene was to be inserted was obtained using the primers of SEQ ID NO: 41 and SEQ ID NO: 42 based on the *Corynebacterium glutamicum* ATCC13869 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 30 seconds, and then polymerization at 72°C for 5 minutes.

The amplified upstream and downstream regions into which the gene was to be inserted, the trpE regulatory region, and the vector pDZ for chromosomal transformation cleaved by *Sma*I restriction enzyme were cloned using the Gibson assembly method to obtain a recombinant plasmid, which was named pDZ-ΔPpheA: :PtrpE. The cloning was performed by mixing the Gibson assembly reagent and each of the gene fragments in a calculated number of moles, followed by incubating at 50°C for 1 hour.

In order to allow the *pheA* gene to be regulated by the promoter of *ilvB* gene, an upstream region into which the gene was to be inserted, the promoter region of *ilvB* gene, and a downstream region into which the gene was to be inserted were obtained. Specifically, a gene fragment in the upstream region into which the gene was to be inserted was obtained using the primers of SEQ ID NO: 37 and SEQ ID NO: 43, a gene fragment in the the promoter region of *ilvB* gene was obtained using the primers of SEQ ID NO: 44 and SEQ ID NO: 45, and a gene fragment in the downstream region into which the gene was to be inserted was obtained using the primers of SEQ ID NO: 46 and SEQ ID NO: 42 based on the *Corynebacterium glutamicum* ATCC13869 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 30 seconds, and then polymerization at 72°C for 5 minutes.

The amplified upstream and downstream regions into which the gene was to be inserted, the promoter region of *ilvB* gene, and the vector pDZ for chromosomal transformation cleaved by *Sma*I restriction enzyme were cloned using the Gibson assembly method to obtain a recombinant plasmid, which was named pDZ-ΔPpheA::PilvB. The cloning was performed by mixing the Gibson assembly reagent and each of the gene fragments in a calculated number of moles, followed by incubating at 50°C for 1 hour.

In order to allow the pheA gene to be regulated by the the promoter of *leuA* gene, an upstream region into which the gene was to be inserted, a leuA promoter region, and a downstream region into which the gene was to be inserted were obtained. Specifically, a gene fragment in the upstream region into which the gene was to be inserted was obtained using the primers of SEQ ID NO: 37 and SEQ ID NO: 47, a gene fragment in the leuA promoter region was obtained using the primers of SEQ ID NO: 48 and SEQ ID NO: 49, and a gene fragment in the downstream region into which the gene was to be inserted was obtained using the primers of SEQ ID NO: 50 and SEQ ID NO: 42 based on the *Corynebacterium glutamicum* ATCC13869 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 30 seconds, and then polymerization at 72°C for 5 minutes.

The amplified upstream and downstream regions in which the gene was to be inserted, the leuA promoter region, and the vector pDZ for chromosomal transformation cleaved by *Sma*I restriction enzyme were cloned using the Gibson assembly method to obtain a recombinant plasmid, which was named pDZ-APpheA::PleuA. The cloning was performed by mixing the Gibson assembly reagent and each of the gene fragments in a calculated number of moles, followed by incubating at 50°C for 1 hour.

In order to allow the *pheA* gene to be regulated by the leuC promoter, an upstream region into which the gene was to be inserted, a leuC promoter region, and a downstream region into which the gene was to be inserted were obtained. Specifically, a gene fragment in the upstream region into which the gene was to be inserted was obtained using the primers of SEQ ID NO: 37 and SEQ ID NO: 51, a gene fragment in the leuC promoter region was obtained using the primers of SEQ ID NO: 52 and SEQ ID NO: 53, and a gene fragment in the downstream region into which the gene was to be inserted was obtained using the primers of SEQ ID NO: 54 and SEQ ID NO: 42 based on the *Corynebacterium glutamicum* ATCC13869 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 30 seconds, and then polymerization at 72°C for 5 minutes.

The amplified upstream and downstream regions into which the gene was to be inserted, the promoter region of *leuC* gene, and the vector pDZ for chromosomal transformation cleaved by *Sma*I restriction enzyme were cloned using the Gibson assembly method to obtain a recombinant plasmid, which was named pDZ-ΔPpheA::PleuC. The cloning was performed by mixing the Gibson assembly reagent and each of the gene fragments in a calculated number of moles, followed by incubating at 50°C for 1 hour.

The constructed pDZ-ΔPpheA::PtrpE, pDZ-ΔPpheA::PilvB, pDZ-ΔPpheA::PleuA, and pDZ-ΔPpheA::PleuC vectors were transformed into the CM06-0003 strain by electroporation and then subjected to a secondary crossover to obtain strains in which the *pheA* gene was allowed to be regulated by the trpE regulatory region or promoter of ilvB, leuA or leuC. The corresponding genetic manipulation was confirmed through genome sequencing and a PCR method using the primers of SEQ ID NO: 55 and SEQ ID NO: 56, which can respectively amplify the external region of the upstream region and downstream region of the homologous recombination where the corresponding regulatory region or promoter was inserted. The resulting strain into which the trpE regulatory region was inserted upstream of the *pheA* was named CM06-0005, the resulting strain into which the ilvB promoter was inserted was named CM06-0006, the resulting strain into which the leuA promoter was inserted was named CM06-0007, and the resulting strain in which the leuC promoter was inserted was named CM06-0008.

Primer sequences used to construct each of the vectors are shown in Table 11 below.

**[Table 11]**

| SEQ ID NO: | Sequence (5'-3') |
|---|---|
| 37 | TTCGAGCTCGGTACCCGGAGGGGTTTCCACCTCG |
| 38 | TGGGAAGCTTGTCTCAATTATGTCTGTTGCTCAATTAGCG |
| 39 | CTAATTGAGCAACAGACATAATTGAGACAAGCTTCCCA |
| 40 | AATTGGTGCGTCGCTCATGGGGCACCTACCGAGGAA |
| 41 | TTCCTCGGTAGGTGCCCCATGAGCGACGCACCAATTGTTG |
| 42 | CGACTCTAGAGGATCCCCCCGAAGAGTTCGGCTGCG |
| 43 | CAGCGCTAATCTTGGCTCTGTCTGTTGCTCAATTAGCG |
| 44 | CTAATTGAGCAACAGACAGAGCCAAGATTAGCGCTGAA |
| 45 | AATTGGTGCGTCGCTCATCCGCTCAGGGGCGGCGG |
| 46 | TCCGCCGCCCCTGAGCGGATGAGCGACGCACCAATTGTTG |
| 47 | GGGGGGAACTTTGGAGTTTGTCTGTTGCTCAATTAGCG |
| 48 | CTAATTGAGCAACAGACAAACTCCAAAGTTCCCCCCC |
| 49 | AATTGGTGCGTCGCTCATTGTGTTCAACCTTCTTAAAAAG |
| 50 | TTAAGAAGGTTGAACACAATGAGCGACGCACCAATTGTTG |
| 51 | AAATGTAAGATTCAAAGATGTCTGTTGCTCAATTAGCG |
| 52 | CTAATTGAGCAACAGACATCTTTGAATCTTACATTTCATAG |
| 53 | AATTGGTGCGTCGCTCATGGAACTCACCGTCCTTAC |
| 54 | GTAAGGACGGTGAGTTCCATGAGCGACGCACCAATTGTTG |
| 55 | CCAGCGATGATCGCGCCG |
| 56 | ATCGCCGTGGAGCCAGCC |

### Example 6: Evaluation of Production Ability of Strains with pheA Gene Promoter Substitution of L-Tyrosine-Producing Strains

In order to confirm the L-tyrosine-producing ability of the strains constructed in Example 5, the strains were cultured using the method and the medium compositions described in Example 2.

**[Table 12]**

| Strain No. | Genotype | Glucos e used (g/L) | Production amount of L-tyrosine (g/L) | Yield of L-tyrosine (%) | Production amount L-phenylalanine (g/L) | Product ion amount L-tryptop han (g/L) |
|---|---|---|---|---|---|---|
| CM06-00 03 | ΔaroP: :Pn-tkt, ΔtyrA: :PgapA-tyrAm, ΔBBD29 _14470::PgapA-aro Gm | 30 | 0.42 | 1.40 | 1.34 | 0.02 |
| CM06-00 04 | CM06-0003 ΔpheA | 13 | 0.66 | 5.08 | 0.04 | 0.02 |
| CM06-00 05 | CM06-0003 APpheA::PtrpE | 30 | 1.61 | 5.37 | 0.03 | 0.02 |
| CM06-00 06 | CM06-0003 ΔPpheA::PilvB | 30 | 0.35 | 1.16 | 1.51 | 0.02 |
| CM06-00 07 | CM06-0003 ΔPpheA::PleuA | 30 | 0.41 | 1.37 | 1.36 | 0.02 |
| CM06-00 08 | CM06-0003 ΔPpheA::PleuC | 30 | 0.48 | 1.61 | 0.89 | 0.02 |

The results of L-tyrosine, L-phenylalanine, and L-tryptophan production in the cultures of L-tyrosine-producing strains CM06-0003, CM06-0004, CM06-0005, CM06-0006, CM06-0007, and CM06-0008 are shown in Table 12 above.

For the CM06-0004 strain in which pheA is deleted, L-tyrosine was produced with a yield of 5.08%. Therefore, in the case of the CM06-0005 strain in which the L-phenylalanine pathway was allowed to be regulated by the L-tryptophan concentration, it produced L-tyrosine while producing L-phenylalanine in a small amount, and thus, a higher yield of L-tyrosine could be expected compared to the CM06-0003 strain in which the L-phenylalanine pathway was not regulated, and a lower yield thereof could be expected compared to the CM06-0004 strain in which the L-phenylalanine pathway was deleted. However, contrary to what was expected, the CM06-0005 strain showed an improved production ability compared to the CM06-0004 strain, thereby producing L-tyrosine with a yield of 5.37%. The L-tyrosine production of CM06-0005 increased by 283% compared to its parent strain CM06-0003 and by 144% compared to the CM06-0004 strain.

From the above results, it can be confirmed that when *pheA* was allowed to be regulated by the L-tryptophan concentration, the L-tyrosine production could be significantly increased to an unexpected level. Additionally, as the control similar to introducing the L-tryptophan regulatory mechanism into *pheA,* the L-tyrosine production was significantly increased compared to the CM06-0006 strain in which the *pheA* was allowed to be regulated by an L-isoleucine concentration, and CM06-0007 and CM06-0008 strains in which the *pheA* was allowed to be regulated by an L-leucine concentration. Therefore, it was confirmed that the introduction of the L-tryptophan regulatory mechanism into *pheA* has a greater synergistic effect on the L-tyrosine production compared to the deletion of *pheA* or the introduction of other regulatory mechanisms.

### Example 7: Construction of non-PTS L-Tyrosine-Producing Strains in which pheA Gene is deleted and Strains in which Promoter is Substituted

Since the L-tyrosine production starts with PEP and E4P as precursors, the use of non-phosphotransferase system (PTS) can allow an enhanced supply of PEP, and thus a high production of L-tyrosine can be expected *(*Nature biotechnol 14 620, 1996). Therefore, *ptsG,* a PTS gene of the strain, was removed, and a *Zymomonas mobilis* ATCC 10988-derived *glf,* a non-PTS gene, was introduced.

In order to delete *ptsG* and insert *glf,* upstream and downstream regions into which the *Zymomonas mobilis-derived glf was* to be inserted were obtained. Specifically, a gene fragment in the upstream region of the *ptsG* gene was obtained using the primers of SEQ ID NO: 57 and SEQ ID NO: 58, and a gene fragment in the downstream region of the *ptsG* gene was obtained using the primers of SEQ ID NO: 59 and SEQ ID NO: 60 based on the *Corynebacterium glutamicum* ATCC13869 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 60 seconds, and then polymerization at 72°C for 5 minutes.

Additionally, in order to obtain the *glf* gene including a well-known cj7 promoter (SEQ ID NO: 61, Korean Patent No. 10-0620092), a cj7 promoter fragment was obtained using the primers of SEQ ID NO: 62 and SEQ ID NO: 63 based on the synthetic cj7 promoter DNA as a template through PCR, and a glf gene fragment was obtained using the primers of SEQ ID NO: 64 and SEQ ID NO: 65 based on the *Zymomonas mobilis* ATCC 10988 chromosomal DNA as a template through PCR. Solg™ Pfu-X DNA polymerase was used as the polymerase, and the PCR was performed under PCR amplification conditions of denaturation at 95°C for 5 minutes, followed by 30 cycles of denaturation at 95°C for 30 seconds, annealing at 60°C for 30 seconds, and polymerization at 72°C for 60 seconds, and then polymerization at 72°C for 5 minutes.

The amplified upstream and downstream regions of the *ptsG* gene, the *glf gene* fragment including the cj7 promoter, and the vector pDZ for chromosomal transformation cleaved by *Sca*I restriction enzyme were cloned using the Gibson assembly method to obtain a recombinant plasmid, which was named pDZ-ΔptsG::pcj7-glf. The cloning was performed by mixing the Gibson assembly reagent and each of the gene fragments in a calculated number of moles, followed by incubating at 50°C for 1 hour.

Primer sequences used in the Example are shown in Table 13 below.

**[Table 13]**

| SEQ ID NO: | Sequence (5'->3') |
|---|---|
| 57 | TTCGAGCTCGGTACCCGAGGGCTCACTGACGTTGA |
| 58 | CGCTGGGATGTTTCTACCGGATTCGATTCCTCAG |
| 59 | CGCTCCCAGAAGTAGGCTCAAACCTTGCCCATAAC |
| 60 | CTCTAGAGGATCCCCCTCCCCCAAACCACGCTTTT |
| 62 | GGAATCGAATCCGGTAGAAACATCCCAGCGCTACT |
| 63 | TACTTTCAGAACTCATGAGTGTTTCCTTTCGTTGG |
| 64 | ACGAAAGGAAACACTCATGAGTTCTGAAAGTAGTC |
| 65 | GGGCAAGGTTTGAGCCTACTTCTGGGAGCGCCACA |

The constructed pDZ-ΔptsG::pcj7-glf vector was transformed into the CM06-0003 strain by electroporation and then subjected to a secondary crossover to obtain a strain into which the *Zymomonas mobilis-derived glf* gene including the cj7 promoter was inserted. The corresponding genetic manipulation was confirmed through genome sequencing and a PCR method using the primers of SEQ ID NO: 66 and SEQ ID NO: 67, which can respectively amplify the external region of the upstream region and downstream region of the homologous recombination where the corresponding gene was inserted, and the resulting strain was named CM06-0009.

**[Table 14]**

| SEQ ID NO: | Sequence (5'-3') |
|---|---|
| 66 | ACATTAAGTGGTAGGCGCTGA |
| 67 | CATAACAGGGCAGAACAAAC |

The pDZ-ΔpheA vector constructed in Example 3 was transformed into the CM06-0009 strain constructed above by electroporation and then subjected to a secondary crossover to obtain a strain in which the *pheA* gene was deleted. The corresponding genetic manipulation was confirmed through genome sequencing and a PCR method using the primers of SEQ ID NO: 35 and SEQ ID NO: 36, which can respectively amplify the external region of the upstream region and downstream region of the homologous recombination where the corresponding gene was deleted, and the resulting strain was named CM06-0011.

The pDZ-ΔPpheA::PtrpE, pDZ-ΔPpheA::PilvB, pDZ-ΔPpheA::PleuA, and pDZ-ΔPpheA::PleuC vectors constructed in Example 5 were each transformed into the CM06-0009 strain constructed above by electroporation and then subjected to a secondary crossover to obtain strains into which each of the regulatory region or promoter was inserted upstream of the *pheA* gene. The corresponding genetic manipulation was confirmed through genome sequencing and a PCR method using the primers of SEQ ID NO: 55 and SEQ ID NO: 56, which can respectively amplify the external region of the upstream region and downstream region of the homologous recombination where the corresponding regulatory region or promoter was inserted. The resulting strain into which the trpE regulatory region was inserted upstream of the *pheA* gene was named CM06-0010, the resulting strain into which the ilvB promoter was inserted was named CM06-0012, the resulting strain into which the leuA promoter was inserted was named CM06-0013, and the resulting strain into which the leuC promoter was inserted was named CM06-0014.

### Example 8: Evaluation of Production Ability of non-PTS L-Tyrosine-Producing Strains in which pheA Gene is Deleted and Strains in which Promoter is Substituted

In order to confirm the L-tyrosine-producing ability of the strains constructed in Example 7, the strains were cultured using the method and the medium composition described in Example 2.

**[Table 15]**

| Strain No. | Genotype | Glucos e used (g/L) | Production amount of L-tyrosine (g/L) | Yield of L-tyrosine (%) | Production amount L-phenylalanine (g/L) | Product ion amount L-tryptop han (g/L) |
|---|---|---|---|---|---|---|
| CM06-00 03 | ΔaroP: :Pn-tkt, ΔtyrA: :PgapA-tyrAm, ΔBBD29 _14470::PgapA-aro Gm | 30 | 0.42 | 1.40 | 1.34 | 0.02 |
| CM06-00 09 | CM06-0003 ΔptsG::pcj7-glf | 30 | 1.46 | 4.86 | 1.67 | 0.03 |
| CM06-00 11 | CM06-0009 ΔpheA | 19 | 1.11 | 5.85 | 0.03 | 0.02 |
| CM06-00 10 | CM06-0009 ΔPpheA::PtrpE | 30 | 2.12 | 7.06 | 0.04 | 0.03 |
| CM06-00 | CM06-0009 ΔPpheA::PilvB | 30 | 1.21 | 4.04 | 2.38 | 0.03 |
| 12 | | | | | | |
| CM06-00 13 | CM06-0009 ΔPpheA::PleuA | 30 | 1.40 | 4.68 | 1.70 | 0.03 |
| CM06-00 14 | CM06-0009 ΔPpheA::PleuC | 30 | 1.68 | 5.60 | 1.11 | 0.03 |

The results of L-tyrosine, L-phenylalanine, and L-tryptophan production in the cultures of L-tyrosine-producing strains CM06-0003, CM06-0009, CM06-0011, CM06-0010, CM06-0012, CM06-0013, and CM06-0014 are shown in Table 15 above.

As expected, it was confirmed that the L-tyrosine and L-phenylalanine productions were increased in CM06-0009, a non-PTS strain capable of enhanced supplying PEP, compared to CM06-0003, which is a PTS strain. Similarly, in this case, no significant increase in L-tryptophan production was observed.

In the case of the CM06-0011 strain in which the *pheA* was deleted based on a non-PTS strain, it was confirmed that although the L-tyrosine production was increased compared to the parent strain with a yield of 5.85%, the final L-tyrosine production amount was decreased compared to the parent strain, and the productivity was reduced due to a significantly low sugar consumption rate. As in Example 6, it could be expected that the CM06-0010 strain could produce L-tyrosine with a lower yield. However, in fact, CM06-0010 produced L-tyrosine with a yield of 7.06%, which was increased by 20.7% compared to the CM06-0011 strain. Additionally, the L-tyrosine production amount of the CM06-0010 strain was increased by 45% compared to its parent strain CM06-0009 and as much as by 91% compared to the CM06-0011 strain. Further, it was also confirmed that the L-tyrosine production was significantly increased as compared to the CM06-00012 strain, in which the *pheA* was allowed to be regulated by an L-isoleucine concentration, and CM06-00013 and CM06-00014 strains, in which the *pheA* was allowed to be regulated by an L-leucine concentration, thereby confirming that the introduction of the L-tryptophan regulatory mechanism into *pheA* has a greater synergistic effect on the L-tyrosine production compared to the deletion of *pheA* or the introduction of other regulatory mechanisms.

### Example 9. Screening of Microorganisms Resistant to L-Tyrosine Analogs

In this Example, in order to release the feedback inhibition by L-tyrosine based on the *Corynebacterium glutamicum* ATCC 13869 as a parent strain, an experiment was performed to select strains resistant to L-tyrosine hydroxamate, which is an L-tyrosine analog, using an artificial mutation method.

Specifically, a mutation was induced by an artificial mutation method using N-methyl-N'-nitro-N-nitrosoguanidine (hereinafter referred to as NTG). The ATCC 13869 strain was cultured in a seed medium for 18 hours and inoculated into 4 mL of the seed medium, and then cultured until OD660 reached about 1.0. After centrifuging the cultured media to recover the cells, the cells were washed twice with 50 mM Tris-malate buffer (pH6.5) and suspended in a final volume of 4 mL of the same buffer. An NTG solution (2 mg/mL in 0.05 M Tris-malate buffer (pH6.5)) was added to the cell suspension to a final concentration of 150 mg/L, and the resultant was allowed to stand at room temperature for 20 minutes and then centrifuged to collect the cells, and the cells were washed twice with the same buffer to remove NTG. The finally washed cells were suspended in 4 mL of a 20% glycerol solution, and stored at -70 C until use. The NTG-treated strains were plated on a minimal medium containing 0.5 g/L of L-tyrosine hydroxamate, and through this process, 100 strains having a resistance to L-tyrosine hydroxamate were obtained.

### Example 10. Evaluation of L-Tyrosine-Producing Ability of Strains having Resistance to L-Tyrosine Hydroxamate

The L-tyrosine-producing ability of the 100 strains of L-tyrosine hydroxamate-resistant strain obtained in Example 9 was confirmed. The 100 strains obtained in Example 9 were each inoculated into a 250-mL corner-baffled flask containing 25 mL of a seed medium and then cultured with shaking at 200 rpm at 30°C for 20 hours. Next, 1 m L of the seed culture solution was inoculated into a 250-mL corner-baffled flask containing 24 mL of a production medium and cultured with shaking at 200 rpm at 30°C for 48 hours.

After the culture, the amount of amino acids produced was measured by HPLC. Among the 100 strains tested, the concentrations of the cultured media of the amino acids for the top 10 strains, which showed an excellent L-tyrosine-producing ability, are shown in Table 16. The 10 candidate strains identified through the above process were named as ATCC 13869 YAR-1 to ATCC 13869 YAR-10, respectively.

Among them, ATCC 13869 YAR-6 and ATCC 13869 YAR-8 strains having the highest L-tyrosine-producing ability were selected.

**[Table 16]**

| **Strain No.** | **Glucose used (g/L)** | **Production amount of L-tyrosine (g/L)** | **Production amount L-phenylalanine (g/L)** | **Production amount L-tryptophan (g/L)** |
|---|---|---|---|---|
| ATCC 13869 | 30 | 0.00 | 0.00 | 0.00 |
| ATCC 13869 YAR-1 | 30 | 0.40 | 0.30 | 0.01 |
| ATCC 13869 YAR-2 | 30 | 0.56 | 0.31 | 0.01 |
| ATCC 13869 YAR-3 | 30 | 0.77 | 0.48 | 0.02 |
| ATCC 13869 YAR-4 | 30 | 0.57 | 0.58 | 0.01 |
| ATCC 13869 YAR-5 | 30 | 0.64 | 0.48 | 0.01 |
| ATCC 13869 YAR-6 | 30 | 0.86 | 0.56 | 0.02 |
| ATCC 13869 YAR-7 | 30 | 0.80 | 0.42 | 0.02 |
| ATCC 13869 YAR-8 | 30 | 0.88 | 0.54 | 0.02 |
| ATCC 13869 YAR-9 | 30 | 0.40 | 0.25 | 0.01 |
| ATCC 13869 YAR-10 | 30 | 0.63 | 0.38 | 0.01 |

### Example 11. pheA Gene Promoter Substitution in Strains Having L-Tyrosine Hydroxamate Resistance

*The pheA* gene of ATCC 13869 YAR-6 and YAR-8 strains selected in Example 10 was allowed to be regulated by the trp operon's regulatory region. To this end, the pDZ-ΔPpheA::PtrpE constructed in Example 5 was transformed into each of the ATCC 13869 YAR-6 and YAR-8 strains by electroporation and then subjected to a secondary crossover to obtain strains in which the *pheA* gene was allowed to be regulated by the trpE regulatory region. The corresponding genetic manipulation was confirmed through genome sequencing and a PCR method using the primers of SEQ ID NO: 55 and SEQ ID NO: 56, which can respectively amplify the external region of the upstream region and downstream region of the homologous recombination where the corresponding regulatory region was inserted. The resulting ATCC 13869 YAR-6 and YAR-8 strains into which the trpE regulatory region was inserted upstream of *the pheA* gene were named as YAR-6P and YAR-8P, respectively.

### Example 12. Evaluation of Production Ability of L-tyrosine Hydroxamate-Resistant ATCC 13869 Strains in which pheA Gene Promoter is Substituted

In order to confirm the L-tyrosine-producing ability of the strains constructed in Example 11, the strains were cultured using the method and the medium composition described in Example 2.

**[Table 17]**

| **Strain No.** | **Glucose used (g/L)** | **Production amount of L-tyrosine (g/L)** | **Yield of L-tyrosine (%)** | **Production amount L-phenylalanine (g/L)** | **Producti on amount L-tryptoph an (g/L)** |
|---|---|---|---|---|---|
| **ATCC 13869** | 30 | 0.00 | 0.00 | 0.00 | 0.00 |
| **ATCC 13869 YAR-6** | 30 | 0.82 | 2.73 | 0.55 | 0.02 |
| **ATCC 13869 YAR-6P** | 30 | 1.50 | 5.00 | 0.04 | 0.02 |
| **ATCC 13869 YAR-8** | 30 | 0.86 | 2.87 | 0.51 | 0.02 |
| **ATCC 13869 YAR-8P** | 30 | 1.52 | 5.07 | 0.03 | 0.02 |

The results of L-tyrosine, L-phenylalanine, and L-tryptophan production in the cultures of the L-tyrosine hydroxamate-resistant ATCC 13869 strains and the strains in which *pheA* gene promoter is substituted are shown in Table 17 above.

As in Examples 6 and 8, when the *pheA* gene is allowed to be regulated by the trpE regulatory region, a decrease in L-phenylalanine production and an increase in L-tyrosine production could be expected. In the actual experimental results, it was confirmed that the ATCC 13869 YAR-6P and ATCC 13869 YAR-8P strains significantly reduced the production of L-phenylalanine as a by-product, and increased the production of L-tyrosine as the target product in a higher level compared to the reduction of L-phenylalanine production. Based on these results, it was confirmed that when the L-tryptophan regulatory mechanism is introduced into *pheA,* the production amount of L-tyrosine could be significantly increased to an unpredictable level without reducing the sugar consumption rate, and that the combination of the trp operon's regulatory region and the *pheA* gene could result in a significant synergistic effect on the L-tyrosine production.

From the above results, it was confirmed that the L-tyrosine production was significantly increased to an unexpected level when *pheA* was allowed to be regulated by the L-tryptophan concentration. In a specific example, it was confirmed that the L-tyrosine production was significantly increased to an unexpected level even in randomly-mutated strains as well as in the strains which have been modified to increase the L-tyrosine-producing ability.

The CM06-0010 strain was deposited at the Korean Culture Center of Microorganisms (KCCM), an International Depositary Authority, under the Budapest Treaty on April, 15, 2019, with Accession No. KCCM12487P.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CM06-0010 | Accession number given by the
INTERNATIONAL DEPOSITARY AUTHORITY:
KCCM12487P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 15. 2019 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 15. 2019.

¹ Where Rule 6.4(d) applies, such date is the date on which the status of international depositary authority was
acquired.

Form BP/4 (sole page)

한국미생물보존센터
[?]
KOREAN CULTURE CENTER OF MICROORGANISMS
[?]

<110>     CJ CheilJedang Corporation

<120>     L-TYROSINE-PRODUCING MICROORGANISM AND METHOD FOR PRODUCING
          L-TYROSINE USING THE SAME

<130>     OPA20084-PCT

<150>     KR 10-2019-0071797
<151>     2019-06-17

<160>     68

<170>     KoPatentIn 3.0

<210>     1
<211>     294
<212>     DNA
<213>     Unknown

<220>
<223>     regulatory regeon of trp operon


<400>     1
taattgagac aagcttccca ctatgtgata aagtcccatt ttgtgaataa ctcttgtctc          60

agtcaaagca cccagtggtg gtggcgcgct aactaagcga gcctgacacc tcaagttgtt         120

ttcactttga tgaatttttt aaggctcgta cttcgttcga cgaagaagcg ggccttttgt         180

ggtttttagc ccacaaccgg caagccctgg atcgaatgaa gctcgcagcg agtaattatt         240

tgatgtttcc cagaaaggct tcagccccac aatgatttcc tcggtaggtg cccc               294


<210>     2
<211>     35
<212>     DNA
<213>     Artificial Sequence

<220>
<223>     primer


<400>     2
tcgagctcgg taccctggga acttgtcgac gctat                                     35


<210>     3
<211>     35
<212>     DNA
<213>     Artificial Sequence

<220>
<223>     primer


<400>     3
tgttcggcaa gcattgtggt gtgggcaatg atcac                                     35


<210>     4
<211>     35

<212>    DNA
<213>    Artificial Sequence

<220>
<223>    primer


<400>    4
attaacggtt aaagtactca ttgtgaggtg gcggg                                35


<210>    5
<211>    35
<212>    DNA
<213>    Artificial Sequence

<220>
<223>    primer


<400>    5
ctctagagga tccccggagc tgctgtccaa cgtgg                                35


<210>    6
<211>    35
<212>    DNA
<213>    Artificial Sequence

<220>
<223>    primer


<400>    6
ccacaccaca atgcttgccg aacatttttc ttttc                                35


<210>    7
<211>    35
<212>    DNA
<213>    Artificial Sequence

<220>
<223>    primer


<400>    7
cacaatgagt actttaaccg ttaatggagt ccttg                                35


<210>    8
<211>    17
<212>    DNA
<213>    Artificial Sequence

<220>
<223>    primer


<400>    8
acgcgccaag tcggacg                                                    17

<210>    9
<211>    19
<212>    DNA
<213>    Artificial Sequence

<220>
<223>    primer


<400>    9
cgcacgatgt ttacctgcg                                                    19


<210>    10
<211>    1122
<212>    DNA
<213>    Unknown

<220>
<223>    tyrA gene


<400>    10
atggttgctg aattgaccgc attacgcgat caaattgatg aagtcgataa agcgctgctg      60

aatttattag cgaagcgtct ggaactggtt gctgaagtgg gcgaggtgaa aagccgcttt     120

ggactgccta tttatgttcc ggagcgcgag gcatctattt tggcctcgcg tcgtgcagag     180

gcggaagctc tgggtgtacc gccagatctg attgaggatg ttttgcgtcg ggtgatgcgt     240

gaatcttact ccagtgaaaa cgacaaagga tttaaaacac tttgtccgtc actgcgtccg     300

gtggttatcg tcggcggtgg cggtcagatg ggacgcctgt tcgagaagat gctgaccctc     360

tcgggttatc aggtgcggat tctggagcaa catgactggg atcgagcggc tgatattgtt     420

gccgatgccg gaatggtgat tgttagtgtg ccaatccacg ttactgagca agttattggc     480

aaattaccgc ctttaccgaa agattgtatt ctggtcgatc tggcatcagt gaaaaatggg     540

ccattacagg ccatgctggt ggcgcatgat ggtccggtgc tggggctaca cccgatgttc     600

ggtccggaca gcggtagcct ggcaaagcaa gttgtggtct ggtgtgatgg acgtaaaccg     660

gaagcatacc aatggtttct ggagcaaatt caggtctggg gcgctcggct gcatcgtatt     720

agcgccgtcg agcacgatca gaatatggcg tttattcagg cactgcgcca ctttgctact     780

tttgcttacg ggctgcacct ggcagaagaa aatgttcagc ttgagcaact tctggcgctc     840

tcttcgccga tttaccgcct tgagctggcg atggtcgggc gactgtttgc tcaggatccg     900

cagctttatg ccgacatcat tatgtcgtca gagcgtaatc tggcgttaat caaacgttac     960

tataagcgtt tcggcgaggc gattgagttg ctggagcagg gcgataagca ggcgtttatt    1020

gacagtttcc gcaaggtgga gcactggttc ggcgattacg tgcagcgttt tcagagtgaa    1080

agccgcgtgt tattgcgtca ggcgaatgac aatcgccagt aa                       1122


<210>    11

<211>    36
<212>    DNA
<213>    Artificial Sequence

<220>
<223>    primer


<400>    11
ttcgagctcg gtaccctatc aaaaccgagt tcttcc                                36


<210>    12
<211>    36
<212>    DNA
<213>    Artificial Sequence

<220>
<223>    primer


<400>    12
gtcgttttta ggcctcctga caagtgtggc acatac                                36


<210>    13
<211>    36
<212>    DNA
<213>    Artificial Sequence

<220>
<223>    primer


<400>    13
tgacaatcgc cagtaatttt atcggctgat gattct                                36


<210>    14
<211>    36
<212>    DNA
<213>    Artificial Sequence

<220>
<223>    primer


<400>    14
actctagagg atccccaacg cgattgcatt cggctc                                36


<210>    15
<211>    36
<212>    DNA
<213>    Artificial Sequence

<220>
<223>    primer


<400>    15
gtgccacact tgtcaggagg cctaaaaacg accgag                                36

<210>    16
<211>    36
<212>    DNA
<213>    Artificial Sequence

<220>
<223>    primer


<400>    16
tcaattcagc aaccatgttg tgtctcctct aaagat                                36


<210>    17
<211>    36
<212>    DNA
<213>    Artificial Sequence

<220>
<223>    primer


<400>    17
ttagaggaga cacaacatgg ttgctgaatt gaccgc                                36


<210>    18
<211>    36
<212>    DNA
<213>    Artificial Sequence

<220>
<223>    primer


<400>    18
tcatcagccg ataaaattac tggcgattgt cattcg                                36


<210>    19
<211>    18
<212>    DNA
<213>    Artificial Sequence

<220>
<223>    primer


<400>    19
gcccactagt cgaatccc                                                    18


<210>    20
<211>    18
<212>    DNA
<213>    Artificial Sequence

<220>
<223>    primer


<400>    20

ctgtccgcaa cctgtgcg                                                    18

<210>    21
<211>    35
<212>    DNA
<213>    Artificial Sequence

<220>
<223>    primer

<400>    21
tcgagctcgg taccccccgg cggtatcgag gtagt                                  35

<210>    22
<211>    35
<212>    DNA
<213>    Artificial Sequence

<220>
<223>    primer

<400>    22
gacaagttta gtactttaat cacccgcggg gaccc                                 35

<210>    23
<211>    35
<212>    DNA
<213>    Artificial Sequence

<220>
<223>    primer

<400>    23
ggtgattaaa gtactaaact tgtcccgagg gtgag                                 35

<210>    24
<211>    35
<212>    DNA
<213>    Artificial Sequence

<220>
<223>    primer

<400>    24
ctctagagga tcccctatca gtcacttccc tgaga                                 35

<210>    25
<211>    35
<212>    DNA
<213>    Artificial Sequence

<220>
<223>    primer

<400>    25
gcgggtgatt aaagtgaggc ctaaaaacga ccgag                                35

<210>    26
<211>    35
<212>    DNA
<213>    Artificial Sequence

<220>
<223>    primer

<400>    26
gttctgataa ttcatgttgt gtctcctcta aagat                                35

<210>    27
<211>    20
<212>    DNA
<213>    Artificial Sequence

<220>
<223>    primer

<400>    27
atgaattatc agaacgacga                                                 20

<210>    28
<211>    35
<212>    DNA
<213>    Artificial Sequence

<220>
<223>    primer

<400>    28
cgggacaagt ttagtttacc cgcgacgcgc tttta                                35

<210>    29
<211>    20
<212>    DNA
<213>    Artificial Sequence

<220>
<223>    primer

<400>    29
ttgatatgac cgcagcctga                                                 20

<210>    30
<211>    20
<212>    DNA
<213>    Artificial Sequence

<220>

<223>    primer

<400>    30
ctgcattctc atcgatcttg                                                    20

<210>    31
<211>    34
<212>    DNA
<213>    Artificial Sequence

<220>
<223>    primer

<400>    31
ttcgagctcg gtacccccga ccaggccaca cgcg                                    34

<210>    32
<211>    40
<212>    DNA
<213>    Artificial Sequence

<220>
<223>    primer

<400>    32
aataatccgg ccggccatgg ggttacacag cttaacccgc                              40

<210>    33
<211>    39
<212>    DNA
<213>    Artificial Sequence

<220>
<223>    primer

<400>    33
cgggttaagc tgtgtaaccc catggccggc cggattatt                               39

<210>    34
<211>    37
<212>    DNA
<213>    Artificial Sequence

<220>
<223>    primer

<400>    34
cgactctaga ggatccccgc tgacaacagc aacgtcg                                 37

<210>    35
<211>    18
<212>    DNA
<213>    Artificial Sequence

<220>
<223>    primer


<400>    35
ccagcgatga tcgcgccg                                                    18


<210>    36
<211>    18
<212>    DNA
<213>    Artificial Sequence

<220>
<223>    primer


<400>    36
atcgccgtgg agccagcc                                                    18


<210>    37
<211>    34
<212>    DNA
<213>    Artificial Sequence

<220>
<223>    primer


<400>    37
ttcgagctcg gtacccggag gggtttccac ctcg                                  34


<210>    38
<211>    40
<212>    DNA
<213>    Artificial Sequence

<220>
<223>    primer


<400>    38
tgggaagctt gtctcaatta tgtctgttgc tcaattagcg                            40


<210>    39
<211>    38
<212>    DNA
<213>    Artificial Sequence

<220>
<223>    primer


<400>    39
ctaattgagc aacagacata attgagacaa gcttccca                              38


<210>    40
<211>    36

<212>    DNA
<213>    Artificial Sequence

<220>
<223>    primer


<400>    40
aattggtgcg tcgctcatgg ggcacctacc gaggaa                                36


<210>    41
<211>    40
<212>    DNA
<213>    Artificial Sequence

<220>
<223>    primer


<400>    41
ttcctcggta ggtgccccat gagcgacgca ccaattgttg                            40


<210>    42
<211>    36
<212>    DNA
<213>    Artificial Sequence

<220>
<223>    primer


<400>    42
cgactctaga ggatcccccc gaagagttcg gctgcg                                36


<210>    43
<211>    38
<212>    DNA
<213>    Artificial Sequence

<220>
<223>    primer


<400>    43
cagcgctaat cttggctctg tctgttgctc aattagcg                              38


<210>    44
<211>    38
<212>    DNA
<213>    Artificial Sequence

<220>
<223>    primer


<400>    44
ctaattgagc aacagacaga gccaagatta gcgctgaa                              38

<210>    45
<211>    35
<212>    DNA
<213>    Artificial Sequence

<220>
<223>    primer


<400>    45
aattggtgcg tcgctcatcc gctcaggggc ggcgg                                35


<210>    46
<211>    40
<212>    DNA
<213>    Artificial Sequence

<220>
<223>    primer


<400>    46
tccgccgccc ctgagcggat gagcgacgca ccaattgttg                           40


<210>    47
<211>    38
<212>    DNA
<213>    Artificial Sequence

<220>
<223>    primer


<400>    47
   ggggggaact ttggagtttg tctgttgctc aattagcg                            38


<210>    48
<211>    37
<212>    DNA
<213>    Artificial Sequence

<220>
<223>    primer


<400>    48
ctaattgagc aacagacaaa ctccaaagtt ccccccc                              37


<210>    49
<211>    40
<212>    DNA
<213>    Artificial Sequence

<220>
<223>    primer


<400>    49
aattggtgcg tcgctcattg tgttcaacct tcttaaaaag                           40

<210>    50
<211>    40
<212>    DNA
<213>    Artificial Sequence

<220>
<223>    primer


<400>    50
ttaagaaggt tgaacacaat gagcgacgca ccaattgttg                              40


<210>    51
<211>    38
<212>    DNA
<213>    Artificial Sequence

<220>
<223>    primer


<400>    51
aaatgtaaga ttcaaagatg tctgttgctc aattagcg                                38


<210>    52
<211>    41
<212>    DNA
<213>    Artificial Sequence

<220>
<223>    primer


<400>    52
ctaattgagc aacagacatc tttgaatctt acatttcata g                            41


<210>    53
<211>    36
<212>    DNA
<213>    Artificial Sequence

<220>
<223>    primer


<400>    53
aattggtgcg tcgctcatgg aactcaccgt ccttac                                  36


<210>    54
<211>    40
<212>    DNA
<213>    Artificial Sequence

<220>
<223>    primer

<400>    54
gtaaggacgg tgagttccat gagcgacgca ccaattgttg                              40


<210>    55
<211>    18
<212>    DNA
<213>    Artificial Sequence

<220>
<223>    primer


<400>    55
ccagcgatga tcgcgccg                                                      18


<210>    56
<211>    18
<212>    DNA
<213>    Artificial Sequence

<220>
<223>    primer


<400>    56
atcgccgtgg agccagcc                                                      18


<210>    57
<211>    35
<212>    DNA
<213>    Artificial Sequence

<220>
<223>    primer


<400>    57
ttcgagctcg gtacccgagg gctcactgac gttga                                   35


<210>    58
<211>    34
<212>    DNA
<213>    Artificial Sequence

<220>
<223>    primer


<400>    58
cgctgggatg tttctaccgg attcgattcc tcag                                    34


<210>    59
<211>    35
<212>    DNA
<213>    Artificial Sequence

<220>
<223>    primer

<400>    59
cgctcccaga agtaggctca aaccttgccc ataac                                35

<210>    60
<211>    35
<212>    DNA
<213>    Artificial Sequence

<220>
<223>    primer

<400>    60
ctctagagga tccccctccc ccaaaccacg ctttt                                35

<210>    61
<211>    318
<212>    DNA
<213>    Unknown

<220>
<223>    cj7 promoter

<400>    61
agaaacatcc cagcgctact aatagggagc gttgaccttc cttccacgga ccggtaatcg      60

gagtgcctaa aaccgcatgc ggcttaggct ccaagatagg ttctgcgcgg ccgggtaatg     120

catcttcttt agcaacaagt tgaggggtag gtgcaaataa gaacgacata gaaatcgtct     180

cctttctgtt tttaatcaac atacaccacc acctaaaaat tccccgacca gcaagttcac     240

agtattcggg cacaatatcg ttgccaaaat attgtttcgg aatatcatgg gatacgtacc     300

caacgaaagg aaacactc                                                   318

<210>    62
<211>    35
<212>    DNA
<213>    Artificial Sequence

<220>
<223>    primer

<400>    62
ggaatcgaat ccggtagaaa catcccagcg ctact                                35

<210>    63
<211>    35
<212>    DNA
<213>    Artificial Sequence

<220>
<223>    primer

<400>    63
tactttcaga actcatgagt gtttcctttc gttgg                                35

<210>    64
<211>    35
<212>    DNA
<213>    Artificial Sequence

<220>
<223>    primer

<400>    64
acgaaaggaa acactcatga gttctgaaag tagtc                                35

<210>    65
<211>    35
<212>    DNA
<213>    Artificial Sequence

<220>
<223>    primer

<400>    65
gggcaaggtt tgagcctact tctgggagcg ccaca                                35

<210>    66
<211>    21
<212>    DNA
<213>    Artificial Sequence

<220>
<223>    primer

<400>    66
acattaagtg gtaggcgctg a                                               21

<210>    67
<211>    20
<212>    DNA
<213>    Artificial Sequence

<220>
<223>    primer

<400>    67
cataacaggg cagaacaaac                                                 20

<210>    68
<211>    1053
<212>    DNA
<213>    Unknown

<220>

<223>    aroG gene

<400>    68
atgaattatc agaacgacga tttacgcatc aaagaaatca aagagttact tcctcctgtc      60

gcattgctgg aaaaattccc cgctactgaa aatgccgcga atacggttgc ccatgcccga     120

aaagcgatcc ataagatcct gaaaggtaat gatgatcgcc tgttggttgt gattggccca     180

tgctcaattc atgatcctgt cgcggcaaaa gagtatgcca ctcgcttgct ggcgctgcgt     240

gaagagctga aagatgagct ggaaatcgta atgcgcgtct attttgaaaa gccgcgtacc     300

acggtgggct ggaaagggct gattaacgat ccgcatatgg ataatagctt ccagatcaac     360

gacggtctgc gtatagcccg taaattgctg cttgatatta acgacagcgg tctgccagcg     420

gcaggtgagt ttctcgatat gatcacccca caatatctcg ctgacctgat gagctggggc     480

gcaattggcg cacgtaccac cgaatcgcag gtgcaccgcg aactggcatc agggctttct     540

tgtccggtcg gcttcaaaaa tggcaccgac ggtacgatta aagtggctat cgatgccatt     600

aatgccgccg gtgcgccgca ctgcttcctg tccgtaacga aatgggggca ttcggcgatt     660

gtgaatacca gcggtaacgg cgattgccat atcattctgc gcggcggtaa agagcctaac     720

tacagcgcga agcacgttgc tgaagtgaaa gaagggctga acaaagcagg cctgccagca     780

caggtgatga tcgatttcag ccatgctaac tcgtccaaac aattcaaaaa gcagatggat     840

gtttgtgctg acgtttgcca gcagattgcc ggtggcgaaa aggccattat tggcgtgatg     900

gtggaaagcc atctggtgga aggcaatcag agcctcgaga gcggggagcc gctggcctac     960

ggtaagagca tcaccgatgc ctgcatcggc tgggaagata ccgatgctct gttacgtcaa    1020

ctggcgaatg cagtaaaagc gcgtcgcggg taa                                 1053

## Claims

1. A L-tyrosine-producing microorganism, comprising a trp operon regulatory region and a gene encoding prephenate dehydratase operably linked thereto.

2. The microorganism of claim 1, wherein the trp operon regulatory region is located upstream of the gene encoding the prephenate dehydratase.

3. The microorganism of claim 1, wherein the gene encoding the prephenate dehydratase is *a pheA* gene.

4. The microorganism of claim 1, wherein the trp operon regulatory region is selected from the group consisting of a trp regulator, a trp promoter, a trp operator, a trp leader peptide, and a trp attenuator.

5. The microorganism of claim 1, wherein the trp operon regulatory region consists of a nucleotide sequence of SEQ ID NO: 1.

6. A method for producing L-tyrosine, comprising culturing the microorganism of any one of claims 1 to 5 in a medium; and recovering L-tyrosine from the cultured microorganism or the medium.

7. An expression cassette comprising a trp operon regulatory region and a gene encoding prephenate dehydratase operably linked thereto.

8. A composition for producing L-tyrosine, comprising a trp operon regulatory region and a gene encoding prephenate dehydratase operably linked thereto, an expression cassette comprising the same, a microorganism comprising the same, or a combination thereof.

9. A method for regulating the activity of prephenate dehydratase using a trp operon regulatory region and a gene encoding prephenate dehydratase operably linked thereto.
